# EUROPEAN PATENT APPLICATION

(11) **EP 2 228 741 A2**
(43) Date of publication of application: **15.09.2010**
(21) Application number: 09012768.9
(22) Date of filing: 08.10.2009
(51) Int. Cl.: G06F 19/00

(54) **Integrated platform for providing contents pertaining to health for chemist's shops, hospitals and clinics, and related process**

(30) Priority: 11.03.2009 IT VI20090053
(71) Applicant: Reggiani, Maria Giovanna, 32043 Cortina D'Ampezzo (BL) (IT)
(72) Inventor: Reggiani, Maria Giovanna, 32043 Cortina D'Ampezzo (IT); Bazzi, Alessandro, 32043 Cortina D'Ampezzo (IT); Gambarotto, Luca, 32043 Cortina D'Ampezzo (IT)
(74) Representative: Garavelli, Paolo

(57) **Abstract**

An integrated platform (1) and a related process are described for providing contents pertaining to health for chemist's shops, hospitals and clinics, comprising: one central server (5) containing an information portal (7) and a data base (11) containing information like health and/or cultural messages and/or videos; at least one local user (3) operatively coupled with the server (5) and adapted to display on a screen (10) such information contained in the data base (11) and transmitted to the user (3) by the portal (7); the integrated platform (1) is adapted to automatically download from the server (5), without any manual intervention by the user (3), the following new pieces of information: an updated application version; an updated version of the configuration file; a new HTML page created by the portal (7); new media files not present by the user (3); and updated media files.

## Description

The present invention refers to an integrated platform for providing contents pertaining to health for chemist's shops, hospitals and clinics, and to a process which is performed using such platform.

The need of spreading information, by pharmaceutical companies and by companies that deal with health in general, inside chemist's shops, is more and more felt. Information that must arrive in these places pertaining to citizens' health are cultural information, which are widely and easily understood by a user, and which promote the health education.

No platform or processes are currently known that enable to promote the health education among citizens using chemist's shops as end-user points.

Object of the present invention is solving the above prior-art problems, by providing an integrated platform and a related process for transmitting health and/or cultural contents to chemist's shops, and more in general to any other health place (hospitals, clinics, etc.).

The above and other objects and advantages of the invention, as will appear from the following description, are obtained by an integrated platform and a related process as claimed in the respective independent Claims. Preferred embodiments and non-trivial variations of the present invention are claimed in the dependent Claims.

The present invention will be better described by some preferred embodiments thereof, given as a non-limiting example, with reference to the enclosed drawings, in which:
- Figure 1 is a schematic diagram showing the main components of the preferred embodiment of the integrated platform of the invention; and
- Figure 2 is a view of a screen appearing as result of the process of the invention.

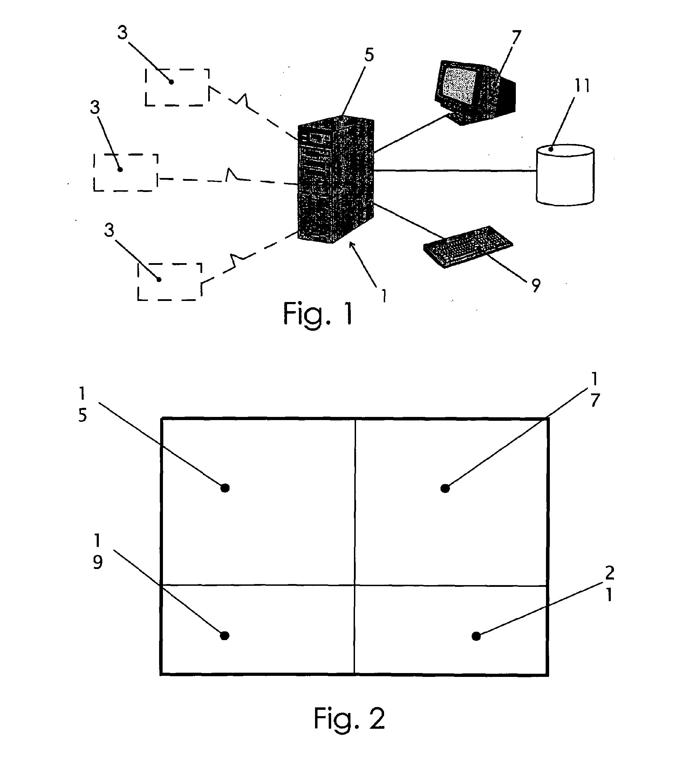

With reference to Fig. 1, the integrated platform 1 of the invention substantially comprises an information portal 7 (schematically shown as a display) containing the communicated messages (screens on the display 7), a central server 5 connected to such display 7, and at least one user 3 (schematically shown as a mini-personal computer placed in a chemist's shop) coupled to the central server 5 (for example through Internet or an Intranet). Usually, a keyboard 9 (for controlling the server 5) and a hard disk 11 (containing the program instructions that enable to perform the process of the invention) are also part of the platform 1.

The mini-personal computers 3, installed in the places where the various communicated messages have to be sent, constantly communicate with the central server 5 and transmit their messages to a video 10, suitably installed and accessible to the public.

Summarising, the present invention allows interfacing the mini-pc 3 located in a chemist's shop with the central server 5 containing the messages to be communicated. The mini-pc 3 on one hand collects a video file and on the other hand transmits the file to the projecting screen 10 for the public. The mini-pc 3 and the server 5 are constantly communicating, for example through an Internet connection, and automatically the server 5 updates the video files that will be transmitted to the video 10 in a chemist's shop.

The server 5 containing advertising and/or health and/or cultural messages will be accessible through an Internet portal through which it will be possible to transmit video and other information to chemist's shops and/or any other public or private health organisation.

The present invention has taken into account all activities to be performed to actuate the transmission of contents on screens 10 accessible to the public, and in particular:
- designing and operating a new system for transmitting multimedia contents;
- updating the functionalities of the portal 7;
- developing a software program that simplifies the transmission of multimedia contents from the server 5 to the mini-pc 3;
- developing an electronic application for communicating and preparing texts;
- performing a technical assistance and maintenance service reserved to the users 3.

The present invention provides that films and other advertising information are present inside a web page 13, directly displayed to the customer through the mini-pc 3 connected to the video 10. The use of Internet technology then introduces a simplification of the mechanism for transmitting films to the public, removing the need by the user 3 to perform download and copy operations. Its objective is providing the user 3 with an all-included service through a "closed and self-installing information box", ready to be automatically downloaded and the local display of films selected by the user 3.

The invention therefore provides a simple and easily usable system in which the user 3 has the chance of choosing the films that he wishes to transmit on his own screen 10, for example in a chemist's shop.

An example that shows how the present electronic platform 1 is simple, for spreading health and cultural contents, for example in chemist's shops, consists in the modes for accessing the product developed by the present invention: the chemist will receive, at the specified address, the necessary hardware for reproducing customised films in his chemist's shop. The operations of installing, connecting and turning-on the system will be directly performed by the chemist, to whom a short installation manual will be supplied.

The system, once turned-on, will be managed through Internet by the customer himself, who will have a reserved access, protected by password: within his personal home-page, the customer will be able to decide what must be transmitted on his screen 10: the mini-pc 3 and the central server 5 will automatically dialogue for transmitting the chosen contents. As anticipated, the use of the Internet technology simplifies the transmission modes for films and other multimedia contents, avoiding that the customer has to perform any manual activity for downloading and copying data.

Through the developed Internet portal 7, the customer will further be able to customise page contents and appearance, creating a real customised "channel" depending on his own needs or his own personal tastes, to be transmitted on the screen 10 visible by his own public.

As regards the "channel" contents, the standard page is divided into four different areas, two of which are available for the chemist, while two are reserved for the system administrator:
- the video area 15 allows showing the
sequence of videos programmed by the customer;
- the text area 17 allows displaying in turn text messages that can be customised by the customer (for example, health prevention and education campaigns, current promotions and offers, offered services, miscellaneous information also of a cultural character); the length of every single message can be customised;
- the data area 19 contains text information set by the system administrator and that cannot be modified by the chemist (for example, chemist's shop opening times, etc.);
- the advertising area 21 contains advertising banners set by the system administrator, that cannot be modified by the customer.

The system allows the customer, limited by his own privileges, to display, inside the various areas, fixed contents (for example the same video or the same text) or to program sequences (for example a sequence of videos, a sequence of texts). Once ended, the programmed sequence is automatically proposed again from the beginning. The modification of the channel contents occurs directly and exclusively through web, by means of a reserved area of the portal 7 protected by login and password. As regards, instead, the page appearance, different possibilities are provided among the customisation options, such as, for example:
- deciding which areas must be displayed (choosing among pre-existing "models")
- deciding arrangement and size of various area (choosing among pre-existing "models")
- deciding background colour, size and colour of the text of each area (choosing among some options, for example bigger text, big text, normal text, small text, smaller text).

The "channel" appearance and contents can be customised by the user 3 without needing training courses, since the platform of the present invention is self-installing and provided in an "all included" mode. All operations are performed inside the reserved area of the portal, protected by a login and password mechanism. The customer will be able to connect himself from home or from any Internet workstation, using a normal connection to the world wide web network. The customisation performed by the user 3 is immediately transmitted on the screen 10 of the mini-pc 3, that self-coordinates itself with the server 5 containing the multimedia contents and the projection screen 7: the web interface on the portal 7 therefore allows the customer to choose the contents that will be transmitted on his own screen 10; the integrated software system makes the mini-pc 3 download the contents chosen on the video 10 by directly taking them from the data base 11 of the server 5.

Other possibilities of customisation at communication level have also been provided, such as for example the chance of creating a sort of "programming", through which video sequences or information messages can be changed depending on the period of the year. After having set a starting date and a final date for a certain area, the new contents will automatically be shown by the system only next to the specified period. Programming can be configured for each individual area. In order to reduce the need of an Internet band for displaying the films, it is necessary that all information related to channel contents and appearance are downloaded, at least the first time, on the mini-pc 3 of the customer. Since all modifications are performed via web, a suitable electronic application has been developed that takes care of the whole synchronisation among information set inside the portal and those stored on the mini-pc 3 and chosen by the customer through his web space. The developed application, integral part of the present invention, is installed on the mini-pc 3: the electronic application will try to connect itself at regular intervals (that can be configured by the system administrator) to the web portal, checking the presence of possible updates. If it is not possible to establish a connection, the mini-pc 3 will repeat the attempt at the following time slot, going on showing, and then transmitting on the screen 10, the previously programmed information to guarantee the service continuity. If the connection is correctly established and updates are available, the application is able to automatically download, without any manual intervention by the customer, all new information such as:
- the updated application version (for example correction of a software error or implementation of a new functionality)
- the updated version of the configuration file (for example if the system administrator has modified the contents synchronisation frequency)
- the new HTML page created by the portal (for
example choice of a different channel "model", modification of colour and size of an area)
- new media files not present on the mini-pc 3 (for example modification of the video sequence by the chemist, modification of advertising messages by the system administrator)
- the updated media files (for example correction of videos by the system administrator after the first publication date).

The connection between the application installed on the mini-pc 3 and the web portal 7 is managed in order to verify the validity of requests and authenticate the customer identity, guaranteeing the privacy of published data and allowing every user 3 to display only data for which he is competent. The present invention has a logic addressed for:
- reducing the used Internet band (locally storing channel-related information and downloading only the contents that have been updated)
- guaranteeing the system operating continuity (in case of problems with the Internet connection, the system goes on operating with the previous programming)
- reducing the need of possible technical
assistance interventions on the mini-pc 3 (the installed software is limited to the operating system, the synchronisation software and a library to display the films, thereby removing the need of a database, an application server and a web server)
- providing a greater control over the system operation and reducing operations care of the mini-pc 3 (the html page is directly generated by the portal 7 and contains therein all necessary information for displaying the channel, as for example programming the sequences).

Moreover, the sequences are composed of the various spots: a user 3 can replace those that he wishes to have by selecting from the video library.

The program can block some videos and no user is able to replace them.

The user has a password that allows him to see and play his own personal films, and the films can be seen by a group of users only and not by others.

## Claims

1. Integrated platform (1) for providing contents pertaining to health for chemist's shops, hospitals and clinics, comprising:
- at least one central server (5) containing an information portal (7) and a data base (11) containing information like health and/or cultural messages and/or videos;
- at least one local user (3) operatively coupled with said central server (5) and adapted to display on a screen (10) said information contained in said data base (11) of said server (5) and transmitted to said user (3) by said information portal (7);
- said integrated platform (1) being adapted to automatically download from the central server (5), without any manual intervention by the user (3), the following new pieces of information:
* an updated application version, such as a correction of a software error or an implementation of a new functionality;
* an updated version of the configuration file;
* a new HTML page created by the portal (7), such as a choice of a different channel model, a modification of colour and a size of an area;
* new media files not present by the user (3), such as a modification of the video sequence by a chemist, a modification of advertising messages by a system administrator; and
* updated media files, such as a correction of videos by the system administrator after the first publication date.

2. Integrated platform (1) according to Claim 1, **characterised in that** the screen (10) contains a html page divided into four different areas, two of which are available for the chemist, while two are reserved for the system administrator:
- a video area (15) adapted to show the sequence of videos programmed by the customer;
- a text area (17) adapted to display in turn text messages that can be customised by the customer, such as health prevention and education campaigns, current promotions and offers, offered services, miscellaneous information also of a cultural character;
- a data area (19) adapted to contain text information set by the system administrator and that cannot be modified by the user (3) such as chemist's shop opening times;
- a advertising area (21) adapted to contain advertising banners set by the system administrator, that cannot be modified by the user (3).

3. Process for providing contents pertaining to health for chemist's shops, hospitals and clinics, said process comprising the steps of:
- constantly sending from a central server (5), containing contents messages to be communicated, to a user (3) video files pertaining to said contents messages;
- transmitting from the user (3) said video files to a projecting screen (10) available for the public; and
- automatically updating from the server (5) the video files that will be transmitted to the screen (10) in a chemist's shop, hospital or clinic.

4. Process according to claim 3, said process further comprising the steps of:
- designing and operating a new system for transmitting multimedia contents;
- updating functionalities of a portal (7) in the central server (5);
- developing a software program that simplifies the transmission of multimedia contents from the server (5) to the user (3);
- developing an electronic application for communicating and preparing texts; and
- performing a technical assistance and maintenance service for the user (3).

5. Process according to claim 3 or 4, said process further comprising the step of:
- logging-in the user (3) to the central server (5) through an access protected by password.

6. Process according to claim 4 or 5, said process further comprising the step of:
- through the developed portal (7), the user (3) customising page contents and appearance, creating a real customised "channel" depending on his own needs or his own personal tastes, to be transmitted on the screen (10) visible by his own public.

7. Process according to any one of claims 3 to 6, said process further comprising, regarding the page appearance, the steps of:
- deciding which areas must be displayed by choosing among pre-existing models;
- deciding arrangement and size of various areas by choosing among pre-existing models; and
- deciding background colour, size and colour of the text of each area by choosing among some options, such as bigger text, big text, normal text, small text, smaller text.

8. Process according to any one of claims 3 to 7, said process further comprising the steps of:
- performing a customisation by the user (3) of his screen (10);
- transmitting the customisation performed by the user (3) to the server (5) containing the multimedia contents and the projection screen (7);
- through a web interface on the portal of the screen (7), allowing the user (3) to choose the contents that will be transmitted on his own screen (10);
- downloading by the user (3) from the server (5) the contents chosen on the video (10) by directly taking them from a data base (11) of the server (5).

9. Process according to any one of claims 3 to 8, said process further comprising the step of:
- creating a programming, through which video sequences or information messages can be changed and displayed depending on the period of the year.

10. Process according to any one of claims 3 to 9, said process further comprising the steps of:
- the user (3) connecting to the server (5) at preset intervals and checking for the presence of possible updates;
- if no updates are present, the user (3) transmitting on the screen (10) the previously programmed information;
- if updates are present, the user (3) automatically downloading all the following updated information:
* an updated application version, such as correction of a software error or implementation of a new functionality;
* an updated version of the configuration file;
* a new HTML page created by the portal, such as choice of a different channel model, modification of colour and size of an area;
* new media files not present by the user (3), such as modification of the video sequence by the chemist, modification of advertising messages by the system administrator; and
* the updated media files, such as correction of videos by the system administrator after the first publication date.

11. Process according to any one of claims 3 to 10, said process further comprising the steps of:
- reducing the used transmission band by locally storing channel-related information and downloading only the contents that have been updated;
- guaranteeing the system operating continuity, so that, in case of problems with the transmission connection, the system goes on operating with the previous programming;
- reducing the need of possible technical assistance interventions on the user (3), since the installed software is limited to the operating system, the synchronisation software and a library to display the films;
- providing a greater control over the system operation and reducing operations care of the user (3), since the html page is directly generated by the portal (7) and contains therein all necessary information for displaying the channel, such as programming the sequences.

12. Process according to any one of claims 3 to 11, said process further comprising the step of:
- the user (3) replacing spots from sequences of information by selecting from the video library from the server (5).

13. Process according to any one of claims 3 to 12, said process further comprising the step of:
- blocking some videos so that no user (3) is able to replace them.
